# EUROPEAN PATENT APPLICATION

(11) **EP 4 095 156 A1**
(43) Date of publication of application: **30.11.2022**
(21) Application number: 21176511.0
(22) Date of filing: 28.05.2021
(51) Int. Cl.: C07K 16/10, A61K 39/12, A61P 31/14

(54) **NEUTRALIZING ANTIBODIES AGAINST HEPATITIS C VIRUS**

(71) Applicant: Universität zu Köln, 50923 Köln (DE)
(72) Inventor: Klein, Florian, 50937 Köln (DE); Weber, Timm Peer Christopher, 50968 Köln (DE); Potthoff, Julian, 51515 Kürten (DE); Pietschmann, Thomas, 30559 Hannover (DE); Bankwitz, Dorothea, 30966 Hemmingen (DE)
(74) Representative: Klöckner, Christoph

(57) **Abstract**

The present invention generally relates to antibodies or antigen-binding fragments thereof against Hepatitis C virus, and such antibodies or antigen-binding fragments thereof for use as a medicament, and in the treatment or prevention of a disease caused by Hepatitis C virus.

## Description

### Technical field

The present invention generally relates to antibodies or antigen-binding fragments thereof against Hepatitis C virus, and such antibodies or antigen-binding fragments thereof for use as a medicament, and in the treatment or prevention of a disease caused by Hepatitis C virus.

### Background of the Invention

HCV infection often remains undiagnosed for decades, but causes progressive liver fibrosis, liver cirrhosis, and hepatocellular carcinoma. Treatment with direct-acting antivirals (DAAs) has reached success rates exceeding 95%. However, owing to the low rate of diagnosis and high treatment costs, only a small percentage of HCV-infected individuals worldwide currently receives DAA treatment. In addition, DAA treatment fails in 3-5% of patients.

Although this is only a small proportion of patients, this problem potentially affects a large number of people worldwide given the high prevalence of chronic HCV infection (approximately 1% of the world's population). It is expected that the use of DAAs against HCV will steadily expand. HCV is a virus with an unusually high ability to mutate. It is thus likely that resistance to available treatments will further increase.

Therefore, it is essential that new treatment options are developed. Monoclonal antibodies have emerged as novel therapeutics for a range of conditions including cancer, autoimmune diseases, and viral infections. They are also a potential novel treatment or prevention option for chronic HCV infection. Indeed, there is evidence that neutralizing antibodies targeting the HCV envelope glycoproteins E1 and E2 can play a protective role in natural HCV infection.

HCV possesses an extraordinary genetic diversity, with 7 major genotypes that differ in 30% of their amino acid sequence on average. A successful antibody treatment or prevention for HCV would therefore have to be broadly neutralizing, ideally against all HCV genotypes.

In natural HCV infection, the main target of neutralizing antibodies seems to be hypervariable region 1 (HVR1) on the E2 protein. HVR1 is characterized by its high genetic variability, even within intra-host populations, and is believed to divert the antibody response away from conserved epitopes such as the CD81 binding site on E2 that is necessary for host cell entry.

In the last years, several antibodies cross-neutralizing multiple HCV genotypes were identified. Knowledge of their epitopes, predominantly located within the CD81 binding site, has helped to guide experimental vaccine design. However, first vaccine trials in chimpanzees and humans have only led to modest results.

We thus need a better understanding of the natural HCV antibody response across different infected individuals, and in particular of specific monoclonal antibodies required for broad neutralization.

Due to the shortcomings mentioned above, there remains a demand for monoclonal human antibodies directed against Hepatitis C virus which have superior neutralization potency and breadth against live virus of different genotypes in comparison to publicly available antibodies.

Thus, it is an object of the present invention to provide novel and advantageous monoclonal antibodies against Hepatitis C virus which have excellent combined neutralization potency and neutralization breadth against live virus of different Hepatitis C virus genotypes. It is a further object of the present invention to provide novel and advantageous monoclonal antibodies against Hepatitis C virus which can be used in treatment or prevention of a disease caused by Hepatitis C virus in human or animal subjects as well as in prevention of infection of a human or animal subject with Hepatitis C virus.

### Summary of the Invention

These objects have been solved by the aspects of the present invention as specified hereinafter.

According to the first aspect of the present invention, an antibody or antigen-binding fragment thereof directed against Hepatitis C virus is provided, wherein the antibody or antigen-binding fragment thereof comprises the heavy chain CDR1 to CDR3 and the light chain CDR1 to CDR3 amino acid sequence of one antibody from the group comprising 1416_01_E03 (with the CDR-H1 of SEQ ID NO: 1, CDR-H2 of SEQ ID NO: 2; CDR-H3 of SEQ ID NO: 3, CDR-L1 of SEQ ID NO: 4, CDR-L2 of SEQ ID NO: 5, and CDR-L2 of SEQ ID NO: 6), 1198_05_G10 (with the CDR-H1 of SEQ ID NO: 7, CDR-H2 of SEQ ID NO: 8; CDR-H3 of SEQ ID NO: 9, CDR-L1 of SEQ ID NO: 10, CDR-L2 of SEQ ID NO: 11, and CDR-L2 of SEQ ID NO: 12), 1382_01_H05 (with the CDR-H1 of SEQ ID NO: 13, CDR-H2 of SEQ ID NO: 14; CDR-H3 of SEQ ID NO: 15, CDR-L1 of SEQ ID NO: 16, CDR-L2 of SEQ ID NO: 17, and CDR-L2 of SEQ ID NO: 18), and 1334_03_A04 (with the CDR-H1 of SEQ ID NO: 19, CDR-H2 of SEQ ID NO: 20; CDR-H3 of SEQ ID NO: 21, CDR-L1 of SEQ ID NO: 22, CDR-L2 of SEQ ID NO: 23, and CDR-L2 of SEQ ID NO: 24). According to a preferred embodiment of the first aspect of the present invention, the antibody or antigen-binding fragment thereof comprises the combination of the variable region heavy chain amino acid sequence and of the variable region light chain amino acid sequence of one antibody selected from the group comprising 1416_01_E03 (with the variable region heavy chain amino acid sequence of SEQ ID NO: 25, and the variable region light chain amino acid sequence of SEQ ID NO: 26), 1198_05_G10 (with the variable region heavy chain amino acid sequence of SEQ ID NO: 27, and the variable region light chain amino acid sequence of SEQ ID NO: 28), 1382_01_H05 (with the variable region heavy chain amino acid sequence of SEQ ID NO: 29, and the variable region light chain amino acid sequence of SEQ ID NO: 30), and 1334_03_A04 (with the variable region heavy chain amino acid sequence of SEQ ID NO: 31, and the variable region light chain amino acid sequence of SEQ ID NO: 32).

According to another preferred embodiment of the first aspect of the present invention, the antibody comprises the combination of the heavy chain sequence and the light chain sequence of one antibody selected from the group comprising 1416_01_E03 (with the heavy chain amino acid sequence of SEQ ID NO: 33, and the light chain amino acid sequence of SEQ ID NO: 34), 1198_05_G10 (with the heavy chain amino acid sequence of SEQ ID NO: 35, and the light chain amino acid sequence of SEQ ID NO: 36), 1382_01_H05 (with the heavy chain amino acid sequence of SEQ ID NO: 37, and the light chain amino acid sequence of SEQ ID NO: 38), and 1334_03_A04 (with the heavy chain amino acid sequence of SEQ ID NO: 39, and the light chain amino acid sequence of SEQ ID NO: 40).

According to one preferred embodiment of the first aspect of the present invention, the CDR sequences, variable region sequences, or heavy chain and light chain sequences are taken from one antibody selected from the group comprising 1416_01_E03, 1198_05_G10, and 1382_01_H05, preferably from one antibody selected from 1416_01_E03 and 1198_05_G10.

According to another preferred embodiment of the first aspect of the present invention, the CDR sequences, variable region sequences, or heavy chain and light chain sequences are taken from the antibody 1416_01_E03.

According to the second aspect of the present invention, a pharmaceutical composition is provided comprising an antibody or antigen-binding fragment thereof according to the first aspect of the present invention, and at least one pharmaceutically acceptable excipient.

According to a preferred embodiment of the second aspect of the present invention, the pharmaceutical composition is a vaccination composition for a human or animal subject, preferably for a human subject.

According to the third aspect of the present invention, a kit is provided comprising an antibody or antigen-binding fragment thereof according to the first aspect of the present invention, and a container.

According to the fourth aspect of the present invention, a nucleic acid is provided encoding an antibody or antigen-binding fragment thereof according to the first aspect of the present invention.

According to the fifth aspect of the present invention, an expression vector is provided comprising a nucleic acid according to the fourth aspect of the present invention.

According to the sixth aspect of the present invention, a host cell is provided comprising a nucleic acid according to the fourth aspect of the present invention or the expression vector according to the fifth aspect of the present invention in functional association with an expression control sequence.

According to the seventh aspect of the present invention, a method of production of an antibody or antigen-binding fragment thereof according to the first aspect of the present invention is provided, comprising cultivating the host cell according to the sixth aspect of the present invention under conditions allowing expression of the antibody or antigen-binding fragment thereof, and recovering the antibody.

According to the eighth aspect of the present invention, an antibody or antigen-binding fragment thereof according to the first aspect of the invention, a pharmaceutical composition according to the second aspect of the invention, a kit according to the third aspect of the invention, a nucleic acid according to the fourth aspect of the invention, or an expression vector according to the fifth aspect of the invention are provided for use as a medicament.

According to the ninth aspect of the present invention, an antibody or antigen-binding fragment thereof according to the first aspect of the invention, a pharmaceutical composition according to the second aspect of the invention, a kit according to the third aspect of the invention, a nucleic acid according to the fourth aspect of the invention, or an expression vector according to the fifth aspect of the invention are provided for use in the treatment or prevention of a disease caused by Hepatitis C virus in human or animal subjects, preferably for use in human subjects. According to the tenth aspect of the present invention, an antibody or antigen-binding fragment thereof according to the first aspect of the invention, a pharmaceutical composition according to the second aspect of the invention, a kit according to the third aspect of the invention, a nucleic acid according to the fourth aspect of the invention, or an expression vector according to the fifth aspect of the invention are provided for use in prevention of infection of a human or animal subject with Hepatitis C virus, preferably for use in human subjects.

According to a preferred embodiment of the ninth or tenth aspect of the present invention, an antibody or antigen-binding fragment thereof according to the first aspect of the invention, a pharmaceutical composition according to the second aspect of the invention, a kit according to the third aspect of the invention, a nucleic acid according to the fourth aspect of the invention, or an expression vector according to the fifth aspect of the invention are provided for use as defined above, wherein the use comprises passive immunization of the human or animal subject.

### Description of Figures

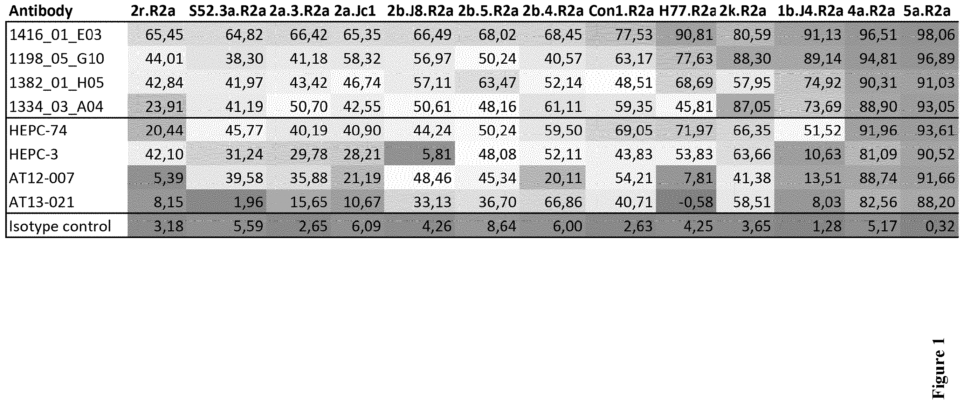

Figure 1 shows the neutralization efficiency of the antibodies of the invention in comparison to antibodies of the prior art against an extended panel of 13 HCVcc strains as described herein and in Bankwitz et al., 2020; HEPC3 and HEPC74 as described in Flyak et al., Cell Host Microbe. 2018 Nov 14;24(5):703-716.e3.doi: 10.1016/j.chom.2018.10.009.; AT12-007 and AT13-021 as described in published international application WO 2016/036252 A2.

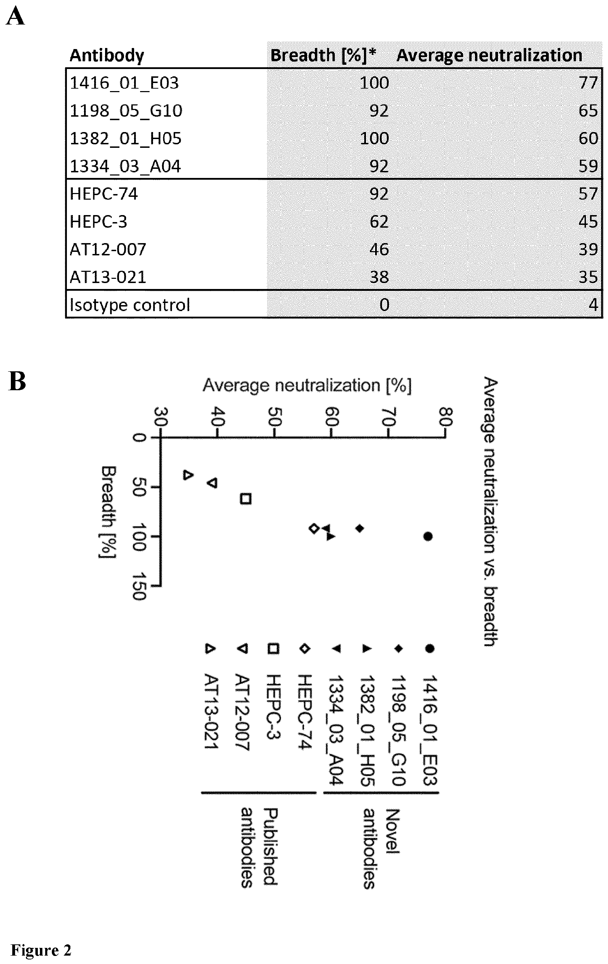

Figure 2 shows the neutralization breadth of the antibodies shown in Figure 1 in terms of percentage of strains neutralized by more than 40%, and in terms of average neutralization efficiency of the neutralized strains in (A) numbers, and in (B) graphic representation.

### Detailed Description of the Invention

The present inventors have dedicated themselves to solving the problem of the present invention and were successful to find novel and useful human monoclonal antibodies against Hepatitis C virus (herein alternatively referenced as HCV) which overcome the disadvantages and shortcomings of known HCV antibodies.

Research on bNAbs targeting Human Immunodeficiency Virus 1 (HIV-1) has previously profited from a strategy where patient cohorts were first screened to identify individuals with broadly neutralizing serum, followed by cloning of antibodies from single antigen-specific B cells obtained from these elite neutralizers (Schommers, P. et al. (2020). Cell 180(3), 471-489).

In an analogous approach, the present inventors now successfully identified HCV elite neutralizers and isolated their HCV-reactive B cells using a fluorescently labeled E2 protein fragment lacking the HVR1 (E2 core). From these cells, novel fully human-origin HCV bNAbs were cloned which for the first time neutralized up to 13 of 13 tested HCV strains covering five of the major HCV genotypes (Bankwitz, D. et al. (2020). Gut gutjnl-2020-321190, and Smith DB et al. (2014) Hepatology;59:318-327.) with superior average neutralization.

Thus, the present invention provides novel and advantageous monoclonal antibodies of human origin with the original pairing of heavy and light chain which are directed against Hepatitis C virus and exceed the neutralization potency and breadth of similar antibodies of the prior art.

Therefore, the inventive antibodies include highly promising candidates for antibody-mediated strategies to effectively treat and prevent Hepatitis C virus infection and disease symptoms caused by such an infection.

The present invention provides antibodies or antigen-binding fragments thereof directed against Hepatitis C virus, wherein the antibody or antigen-binding fragment thereof comprises the heavy chain CDR1 to CDR3 and the light chain CDR1 to CDR3 amino acid sequence of one antibody from the group comprising 1416_01_E03 (with the CDR-H1 of SEQ ID NO: 1, CDR-H2 of SEQ ID NO: 2; CDR-H3 of SEQ ID NO: 3, CDR-L1 of SEQ ID NO: 4, CDR-L2 of SEQ ID NO: 5, and CDR-L2 of SEQ ID NO: 6), 1198_05_G10 (with the CDR-H1 of SEQ ID NO: 7, CDR-H2 of SEQ ID NO: 8; CDR-H3 of SEQ ID NO: 9, CDR-L1 of SEQ ID NO: 10, CDR-L2 of SEQ ID NO: 11, and CDR-L2 of SEQ ID NO: 12), 1382_01_H05 (with the CDR-H1 of SEQ ID NO: 13, CDR-H2 of SEQ ID NO: 14; CDR-H3 of SEQ ID NO: 15, CDR-L1 of SEQ ID NO: 16, CDR-L2 of SEQ ID NO: 17, and CDR-L2 of SEQ ID NO: 18), and 1334_03_A04 (with the CDR-H1 of SEQ ID NO: 19, CDR-H2 of SEQ ID NO: 20; CDR-H3 of SEQ ID NO: 21, CDR-L1 of SEQ ID NO: 22, CDR-L2 of SEQ ID NO: 23, and CDR-L2 of SEQ ID NO: 24).

Within the context of the present invention, the antibodies, which have been generated and described herein, may be used and claimed as the complete monoclonal human antibody or as any functional or antigen-binding fragment thereof. Preferably, the antibody or any kind of functional or antigen-binding fragment thereof should at least comprise the complementarity determining regions (CDR) 1 to 3 of the heavy chain and CDR 1 to 3 of the light chain of the antibody.

The CDR regions of the antibody sequences described herein are preferably defined according to the numbering scheme of IMGT which is an adaptation of the numbering scheme of Chothia (ImMunoGeneTics information system^{®}; Lefranc et al., NAR 27: 209-212 (1999); http://imgt.cines.fr).

According to one preferred embodiment of the present invention, the CDR sequences of the light and heavy chain sequences of the antibodies described herein are as follows:

| **Comprised in SEQ ID NO:** | **Source** | **CDR1** | **SEQ ID of CDR1** | **CDR2** | **SEQ ID of CDR2** | **CDR3** | **SEQ ID of CDR3** |
|---|---|---|---|---|---|---|---|
| 25 (variable region) / 33 (full length) | Heavy chain of 1416_01_E03 | GGTFSR FG | 1 | IIPKFGT T | 2 | AREVAP SNSFFL SWFDP | 3 |
| 26 (variable region) / 34 (full length) | Light chain of 1416_01_E03 | SSDIGSY NL | 4 | EVT | 5 | CSYAR GSTYWI | 6 |
| 27 (variable region) / 35 (full length) | Heavy chain of 1198_05_G10 | GGTVNT | 7 | IFPLLGV P | 8 | AKDGV GWSGH GPSQW SGVDV | 9 |
| 28 (variable region) / 36 (full length) | Light chain of 1198_05_G10 | QSLLHST GYNY | 10 | LGS | 11 | MQALEI PRLT | 12 |
| 29 (variable region) / 37 (full length) | Heavy chain of 1382_01_H05 | GGSFGR DA | 13 | IIPIFGTS | 14 | VRWSF GDYGLF LTEGDY | 15 |
| 30 (variable region) / 38 (full length) | Light chain of 1382_01_H05 | QSVDKY | 16 | ETS | 17 | HHRGN WPPSFT | 18 |
| 31 (variable region) / 39 (full length) | Heavy chain of 1334_03_A04 | GGTFRS YT | 19 | IISVGNS A | 20 | ARDGN FDWRT ARNEQ NWFDP | 21 |
| 32 (variable region) / 40 (full length) | Light chain of 1334_03_A04 | QTIGNF | 22 | GAS | 23 | QQTYN SPRVT | 24 |

Based on the common general knowledge and the information given herein on the variable region heavy chain amino acid sequences and the variable region light chain amino acid sequences of the antibodies of the invention, the CDRs may be easily and unambiguously derived from the amino acid sequences of the heavy and light chains or the variable regions thereof by a skilled person.

In the following, the full length heavy and light chain amino acid sequences as well as the variable regions thereof of the antibodies of the invention are disclosed in one-letter code:

### Antibody 1416 01 E03 (variable regions in bold characters):

### Heavy chain (full length as SEQ ID NO: 33; variable region as SEQ ID NO: 25)

MGWSCIILFLVATATGVHSQVQLVQSGAEVKKPGSSVKVSCKASGGTFSRFGISWVRQAPGQ
GLEWMGGIIPKFGTTNYAQKFQGRVTITADKSTNIAYLEVSSLTSEDTAVYYCAREVAPSNSFF
LSWFDPWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKRVEPKSCDKTHTC
PPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLP
PSREEMTKNQVSLTCLVKGFYPSDIAVEWESNGQPENNYKTTPPVLDSDGSFFLYSKLTVDKS
RWQQGNVFSCSVMHEALHNHYTQKSLSLSPGK

### Light chain (full length as SEQ ID NO: 34: variable region as SEQ ID NO: 26)

MGWSCIILFLVATATGSWAQSALTQPASVSGSPGQSITISCTGTSSDIGSYNLVSWYQQHPGK
APKLMISEVTERPSGVSARFSGSKSGNTASLTISGLQAEDEADYFCCSYARGSTYWIFGGGT
KLTVLGQPKAAPSVTLFPPPSEELQANKATLVCLISDFYPGAVTVAWKADSSPVKAGVETTTPS
KQSNNKYAASSYLSLTPEQWKSHRSYSCQVTHEGSTVEKTVAPTECS

### Antibody 1198 05 G10 (variable regions in bold characters):

### Heavy chain (full length as SEQ ID NO: 35: variable region as SEQ ID NO: 27)

MGWSCIILFLVATATGVHSQVQLVQSGAEVKKPGSSVKVSCRTSGGTVNTLHWVRQAPGQG
LEWMGSIFPLLGVPTYAQKFQGRVTVTADRSTSTAYMELRSLRADDTAVYYCAKDGVGWSG
HGPSQWSGVDVWGPGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVDKRVEPKSC
DKTHTCPPCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLDSDGSFFLYSK
LTVDKSRWQQGNVFSCSVMHEALHNHYTQKSLSLSPGK

### Light chain (full length as SEQ ID NO: 36; variable region as SEQ ID NO: 28)

MGWSCIILFLVATATGVHSDIVMTQSPLSLPVTPGEPASISCTSSQSLLHSTGYNYLDWYVQKP
GQSPQLLIYLGSIRASGVPDRFSGSGSGTDFTLRISRVEAGDVGIYYCMQALEIPRLTFGGGTK
LEIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC

### Antibody 1382 01 H05 (variable regions in bold characters):

### Heavy chain (full length as SEQ ID NO: 37; variable region as SEQ ID NO: 29)

MGWSCIILFLVATATGVHSQVQLVQSGAEVKKPGSSVKVSCTTSGGSFGRDAVSWVRQAPG
QGLEPMGGIIPIFGTSSYAQTFQGRVTFTAGKSTSTAYMELSSLKSEDTAVYYCVRWSFGDYG
LFLTEGDYWGQGTLVIVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKRVEPKSCDKTHT
CPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLP
PSREEMTKNQVSLTCLVKGFYPSDIAVEWESNGQPENNYKTTPPVLDSDGSFFLYSKLTVDKS
RWQQGNVFSCSVMHEALHNHYTQKSLSLSPGK

### Light chain (full length as SEQ ID NO: 38; variable region as SEQ ID NO: 30)

MGWSCIILFLVATATGVHSEIVLTQSPASLSLSPGERATLSCRASQSVDKYFAWYQQKPGQAP
RLLIYETSKRATGIPARFSGSGSGTDFTLTISNLEPDDFAIYYCHHRGNWPPSFTFGQGTRLEI
KRTVAAPSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC

### Antibody 1334 03 A04 (variable regions in bold characters):

### Heavy chain (full length as SEQ ID NO: 39; variable region as SEQ ID NO: 31)

MGWSCIILFLVATATGVHSQVQLVQSGAEVKKPGSSVKVSCKASGGTFRSYTFSWVRQAPG
HGLEWMGGIISVGNSATYAQKFQGRVTITADKSTSTVYMELSGLTSEDTAIYFCARDGNFDW
RTARNEQNWFDPWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKAKGQPRE
PQVYTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

### Light chain (full length as SEQ ID NO: 40; variable region as SEQ ID NO: 32)

MGWSCIILFLVATATGVHSDIQMTQSPSSLSASVGDRVTLTCRASQTIGNFLNWYQQKPGRAP
KLLVYGASNLQSGVPSRFSGSGSGTDFTLTISSLEPEDFATYYCQQTYNSPRVTFGQGTRLDI

KRTVAAPSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC

According to one preferred embodiment, the present invention also provides antibodies or antigen-binding fragments thereof, wherein the antibody or antigen-binding fragment thereof comprises the combination of the variable region heavy chain amino acid sequence and of the variable region light chain amino acid sequence of one antibody selected from the group comprising 1416_01_E03 (with the variable region heavy chain amino acid sequence of SEQ ID NO: 25, and the variable region light chain amino acid sequence of SEQ ID NO: 26), 1198_05_G10 (with the variable region heavy chain amino acid sequence of SEQ ID NO: 27, and the variable region light chain amino acid sequence of SEQ ID NO: 28), 1382_01_H05 (with the variable region heavy chain amino acid sequence of SEQ ID NO: 29, and the variable region light chain amino acid sequence of SEQ ID NO: 30), and 1334_03_A04 (with the variable region heavy chain amino acid sequence of SEQ ID NO: 31, and the variable region light chain amino acid sequence of SEQ ID NO: 32).

According to another preferred embodiment, the present invention also provides antibodies or antigen-binding fragments thereof, wherein the antibody comprises the combination of the heavy chain sequence and the light chain sequence of one antibody selected from the group comprising 1416_01_E03 (with the heavy chain amino acid sequence of SEQ ID NO: 33, and the light chain amino acid sequence of SEQ ID NO: 34), 1198_05_G10 (with the heavy chain amino acid sequence of SEQ ID NO: 35, and the light chain amino acid sequence of SEQ ID NO: 36), 1382_01_H05 (with the heavy chain amino acid sequence of SEQ ID NO: 37, and the light chain amino acid sequence of SEQ ID NO: 38), and 1334_03_A04 (with the heavy chain amino acid sequence of SEQ ID NO: 39, and the light chain amino acid sequence of SEQ ID NO: 40).

According to a preferred embodiment of the present invention, the CDR sequences, variable region sequences, or heavy chain and light chain sequences are taken from one antibody selected from the group comprising the antibodies disclosed herein as 1416_01_E03, 1198_05_G10, and 1382_01_H05, preferably from one antibody selected from 1416_01_E03 and 1198_05_G10.

According to another preferred embodiment of the present invention, the CDR sequences, variable region sequences, or heavy chain and light chain sequences are taken from the antibody disclosed and referenced herein as 1416_01_E03.

In general, the antibodies or antigen-binding fragments thereof as described herein further encompass antibody amino acid sequences being at least 80% identical to the sequences as defined above as long as they are still binding to the core domain comprising amino acids 412 to 645 of the E2 glycoprotein of Hepatitis C virus of genotype 2b (with the amino acid sequence of SEQ ID NO: 41).

This is meant to include sequences having trivial mutations of the antibody amino acid sequence which do not interfere with structural folds and the affinity of the antibody to the E2 glycoprotein. Preferably, the deviations in the amino acid sequence leading to an at least 80%, 85%, 90% or 95% overall identity to the individualized sequences explicitly disclosed herein are present exclusively outside the CDR regions of the antibodies according to the invention, more preferably the deviations are present exclusively outside the variable regions of the light and heavy chains.

The antibodies according to the present invention are preferably of human origin. Thus, at least the sequences outside the CDRs, such as framework and constant regions of the antibody, are preferably of human origin or can be attributed to human origin. Furthermore, the antibodies of the present invention are preferably monoclonal.

In one preferred embodiment, the antibody is a monoclonal antibody or a fragment thereof that retains binding specificity and ability to neutralize infectious pathogen. In one preferred embodiment, the antibody is an IgG1, IgG2, IgG3, or IgG4 antibody. For example, the antibody may be an antibody comprising an Fc domain of any human IgG isotype (e.g. IgG1, IgG2, IgG3, or IgG4).

Optionally, the antigen-binding fragment consists of or comprises a Fab, Fab', Fab'-SH, F(ab)₂, F(ab')₂, Fv (typically the VL and VH domains of a single arm of an antibody), single-chain Fv (scFv; see, e.g., Bird et al., Science 242:42S-426 (1988); Huston et al., PNAS 85: 5879-5883 (1988)), dsFv, Fd (typically the VH and CH1 domain), and dAb (typically a V_{H} domain) fragments; V_{H}, V_{L}, V_{HH}, and V-NAR domains; monovalent molecules comprising a single V_{H} and a single V_{L} chain; minibodies, diabodies, triabodies, tetrabodies, and kappa bodies (see, e.g., III et al., Protein Eng 10:949-57 (1997)); camel IgG; IgNAR; as well as one or more isolated CDRs or a functional paratope, where the isolated CDRs or antigen-binding residues or polypeptides can be associated or linked together so as to form a functional antibody fragment.

Various types of antibody fragments have been described or reviewed in, e.g., Holliger and Hudson, Nat Biotechnol 2S:1126-1136 (2005); International Publ. No. WO 2005/040219, and U.S. Publ. Nos. 2005/0238646 and 2002/0161201. These antibody fragments may be obtained using conventional techniques known to those of skill in the art, and the fragments may be screened for utility in the same manner as intact antibodies.

Within the present invention, an antibody or antigen-binding fragment thereof directed against Hepatitis C virus may preferably mean an antibody or antigen-binding fragment thereof binding to the core domain comprising amino acids 412 to 645 of the E2 glycoprotein of Hepatitis C virus of genotype 2b (with the amino acid sequence of SEQ ID NO: 41), more preferably with an at least 10-fold, even more preferably at least 50-fold, particularly preferably at least 100-fold increased affinity compared to unrelated epitopes, proteins or protein regions.

According to one preferred embodiment of the present invention, the antibody or antigen-binding fragment thereof exhibits a binding constant (K_{D}) to the E2 core domain of SEQ ID NO: 41 as determined by surface plasmon resonance of 20 nM or less, preferably of 5 nM or less, more preferably of 1 nM or less, even more preferably of 0.2 nM or less, particularly preferably of 0.1 nM or less.

It is a trivial task for a skilled person to determine if an antibody which exhibits a certain degree of identity is still directed against and binding to the core domain comprising the amino acid sequence of SEQ ID NO: 41 based on the above or the common general knowledge.

The determination of percent identity between two sequences is accomplished according to the present invention by using the mathematical algorithm of Karlin and Altschul (Proc. Natl. Acad. Sci. USA (1993) 90: 5873-5877). Such an algorithm is the basis of the BLASTN and BLASTP programs of Altschul et al. (J. Mol. Biol. (1990) 215: 403-410). BLAST nucleotide searches are performed with the BLASTN program. To obtain gapped alignments for comparative purposes, Gapped BLAST is utilized as described by Altschul et al. (Nucleic Acids Res. (1997) 25: 3389-3402). When utilizing BLAST and Gapped BLAST programs, the default parameters of the respective programs are used.

According to a preferred embodiment of the present invention, antibody amino acid sequences form part of the invention which consist of or comprise a nucleic acid sequence being at least 85% identical to the sequences defined above and disclosed herein, more preferably at least 90% identical, even more preferred at least 95% identical.

According to a preferred embodiment of the present invention, the Hepatitis C virus strains encompassed and targeted by the antibodies or antigen-binding fragments thereof according to the present invention are any of the seven major genotypes as disclosed in Smith DB et al. (2014) Hepatology;59:318-327. which may collectively be referenced herein as Hepatitis C virus.

According to another preferred embodiment of the present invention, the Hepatitis C virus strains which are targeted are one or more of the genotypes 1, 2, 3, 4 and 5. However, preferably, the antibody or antigen-binding fragment thereof should also be directed against equivalent sequences of other virus variants or strains.

According to one preferred embodiment of the present invention, the antibody or antigen-binding fragment thereof exhibits a neutralization potency of at least 40% inhibition at 50 µg/ml of the strain of genotype 2a (J6/JFH1), preferably of all 13 HCVcc strains (2r, 3a, 2a, 2a-3, 2b, 2b-4, 2b-5, 1b con, 1a, 1b J4, 2k, 5a, and 4a) used in the neutralization assay disclosed herein.

Said assay may preferably be carried out as disclosed in Koutsoudakis, G et al. (2006). J. Virol. 80, 5308-5320. and/or in Wakita, T. et al. (2005). Nat. Med. 11, 791-796. The HCV strains used may include or consist of one or more, or all of 13 renilla luciferase reporter HCVcc strains (2r, 3a, 2a, 2a-3, 2b, 2b-4, 2b-5, 1b con, 1a, 1b J4, 2k, 5a, and 4a) as described herein and in Bankwitz, D. et al. (2020). Gut gutjnl-2020-321190.

In the description of the present application, internal antibody designations may be used. It is pointed out that the antibodies consist of heavy and light chains which also form part of the present description. If reference is made to an antibody by its internal designation or to a SEQ ID NO:, it should be understood that these ways of reference are interchangeable.

The present invention further relates to a pharmaceutical composition comprising an antibody or antigen-binding fragment thereof according to the invention as defined and further described herein and at least one pharmaceutically acceptable excipient. Preferably, the pharmaceutical composition is a vaccination composition for a human or animal subject, preferably for a human subject.

As used herein, "vaccination composition" means a pharmaceutical composition comprising at least one antibody or antigen-binding portion thereof of the present invention which is capable of providing passive immunity. "Passive immunity" as used herein means supplementing a subject's immune response to an antigen or pathogen by providing antibodies and/or antigen-binding portions thereof which neutralize an antigen. In a preferred embodiment of the present invention, the pharmaceutical composition is a passive immunization composition and/or provides passive immunity to the subject.

The present invention also encompasses a kit comprising an antibody or antigen-binding fragment thereof according to the invention as defined and further described herein and a container.

The present invention further discloses a nucleic acid encoding an antibody or antigen-binding fragment thereof according to the present invention. Also, the present invention provides an expression vector comprising a nucleic acid according to the present invention in functional association with an expression control sequence.

The nucleic acids can be present in whole cells, in a cell lysate, or in a partially purified or substantially pure form. A nucleic acid is "isolated" or "rendered substantially pure" when purified away from other cellular components or other contaminants, e.g., other cellular nucleic acids (e.g., the other parts of the chromosome) or proteins, by standard techniques, including alkaline/SDS treatment, CsCI banding, column chromatography, agarose gel electrophoresis and others well known in the art. See, F. Ausubel, et al., ed. Current Protocols in Molecular Biology, Greene Publishing and Wiley Interscience, New York (1987).

Nucleic acids, e.g., cDNA, can be mutated, in accordance with standard techniques to provide gene sequences. For coding sequences, these mutations, can affect amino acid sequence as desired. In particular, DNA sequences substantially homologous to or derived from native V, D, J, constant, switches and other such sequences described herein are contemplated (where "derived" indicates that a sequence is identical or modified from another sequence).

The term "vector," as used herein, is intended to refer to a nucleic acid molecule capable of transporting another nucleic acid to which it has been linked. One type of vector is a "plasmid," which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors).

Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked.

Such vectors are referred to herein as "recombinant expression vectors" (or simply, "expression vectors") In general, expression vectors of utility in recombinant DNA techniques are often in the form of plasmids. In the present specification, "plasmid" and "vector" can be used interchangeably as the plasmid is the most commonly used form of vector. However, also included are other forms of expression vectors, such as viral vectors (e.g., replication defective retroviruses, adenoviruses and adeno-associated viruses), which serve equivalent functions.

The present invention further relates to a host cell comprising a nucleic acid according to the present invention, or the expression vector according to the present invention.

The term "recombinant host cell" (or simply "host cell"), as used herein, is intended to refer to a cell that comprises a nucleic acid that is not naturally present in the cell, and can be a cell into which a recombinant expression vector has been introduced. It should be understood that such terms are intended to refer not only to the particular subject cell but to the progeny of such a cell. Because certain modifications can occur in succeeding generations due to either mutation or environmental influences, such progeny cannot, in fact, be identical to the parent cell, but are still included within the scope of the term "host cell" as used herein.

In one aspect, the present invention is also directed to a method of production of an antibody or antigen-binding fragment thereof of the present invention, comprising cultivating the host cell of the present invention under conditions allowing expression of the antibody or antigen-binding fragment thereof, and recovering the antibody.

The present invention is also directed to the antibody or antigen-binding fragment thereof according to the invention as defined and further described herein, the pharmaceutical composition as described herein, the kit as described herein, a nucleic acid as described herein, or an expression vector as described herein for use as a medicament.

In another aspect, the present invention is also directed to the antibody or antigen-binding fragment thereof according to the invention as defined and further described herein, the pharmaceutical composition as described herein, the kit as described herein, a nucleic acid as described herein, or an expression vector as described herein for use in the treatment or prevention of a disease caused by Hepatitis C virus in human or animal subjects, preferably for use in human subjects.

In one aspect, the present invention is directed to the antibody or antigen-binding fragment thereof according to the invention as defined and further described herein, the pharmaceutical composition as described herein, the kit as described herein, a nucleic acid as described herein, or an expression vector as described herein for use in prevention of infection of a human or animal subject with Hepatitis C virus, preferably of infection of a human subject with Hepatitis C virus.

Within the uses of the antibodies or antigen-binding fragments thereof in human or animal subjects as disclosed herein, the use preferably comprises passive immunization of the human or animal subject.

In one embodiment of the present invention, an antibody or antigen-binding fragment thereof according to the invention is administered to a patient in need thereof by intravenous injection or infusion. In a preferred embodiment, the antibody is administered by intravenous infusion at a dose of 1 mg/kg body weight to 100 mg/kg body weight of the patient. In a preferred embodiment, the antibody is administered at a dose of 2,5 mg/kg, 5 mg/kg, 10 mg/kg, 20 mg/kg, 25 mg/kg, 30 mg/kg, 40 mg/kg, 50 mg/kg, or 100 mg/kg. The dosage of an antibody or an antigen-binding fragment thereof of the invention to be administered to a subject can further vary depending on such things as the severity of the symptoms exhibited as well as the age, sex, and health of the subject.

A pharmaceutical composition of the invention is formulated to be compatible with its intended route of administration. Examples of routes of administration include, but are not limited to, parenteral, e.g., intravenous, intradermal, subcutaneous, oral, intranasal (e.g., inhalation and inhaled through the mouth), transdermal (e.g., topical), transmucosal, and rectal administration.

In a specific embodiment, the composition is formulated in accordance with routine procedures as a pharmaceutical composition adapted for intravenous, subcutaneous, intramuscular, oral, intranasal, or topical administration to human beings. Typically, compositions for intravenous administration are solutions in sterile isotonic aqueous buffer. Where necessary, the composition may also include a solubilizing agent and a local anesthetic such as lignocaine to ease pain at the site of the injection.

The methods of the invention may also comprise administration of a composition formulated for parenteral administration by injection (e.g., by bolus injection or continuous infusion).

Formulations for injection may be presented in unit dosage form (e.g., in ampoules or in multidose containers) with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle (e.g., sterile pyrogen-free water) before use.

The methods of the invention may additionally comprise of administration of compositions formulated as depot preparations. Such long acting formulations may be administered by implantation (e.g., subcutaneously or intramuscularly) or by intramuscular injection. Thus, for example, the compositions may be formulated with suitable polymeric or hydrophobic materials (e.g., as an emulsion in an acceptable oil) or ion exchange resins, or as sparingly soluble derivatives (e.g., as a sparingly soluble salt).

The pharmaceutical formulation of the present invention may be provided in liquid form or may be provided in lyophilized form.

The pharmaceutical formulation according to the present invention may comprise a buffering agent. Buffering agents include, but are not limited to citric acid, HEPES, histidine, potassium acetate, potassium citrate, potassium phosphate (KH2PO4), sodium acetate, sodium bicarbonate, sodium citrate, sodium phosphate (NAH2PO4). Tris base, and Tris-HCl. In one embodiment, the buffering agent is histidine. In certain embodiments, the histidine concentration is about 5, 10, 15, 20, 25, 30, 35, 40, 45, 50, 55, 60, 65, 70, 75, 80, 85, 90, 95, or 100 mM. In one embodiment the histidine concentration is 10.+-.5 mM. In one embodiment, the histidine concentration is 10-2 mM. In one embodiment, the histidine concentration is about 10 mM. In one embodiment, the histidine concentration is about 15 mM.

The buffer used in the formulations in accordance with the present invention may have a pH in the range from about 5.5 to about 7.5, or from about 5.8 to about 7.0. In one embodiment the pH is about 6.0. In one embodiment the pH is about 7.0. Examples of buffering agents that will control the pH in this range include acetate, succinate, gluconate, histidine, citrate, glycylglycine and other organic acid buffers.

The pharmaceutical formulation according to the present invention may comprise a tonicity agent. Tonicity agents, include, but are not limited to dextrose, glycerin, mannitol, potassium chloride, and sodium chloride. In one embodiment the tonicity agent is sodium chloride. In one embodiment the sodium chloride concentration is about 70 to 170 mM; about 90-150 mM; or about 115.+-.10 mM. In certain embodiments the sodium chloride concentration is about 70, 75, 80, 85, 90, 95, 100, 105, 110, 115, 120, 125, 130, 135, 140, 145, 150, 155, 160, 165, 170, or 175 mM. In one embodiment, the sodium chloride concentration is about 115 mM. In another embodiment, the sodium chloride concentration is 150.+-.10 mM. In one embodiment, the sodium chloride concentration is about 150 mM.

In certain embodiments, the pharmaceutical formulation according to the present invention comprises a stabilizer. Stabilizers, include, but are not limited to human serum albumin (hsa), bovine serum albumin (bsa), .alpha.-casein, globulins, .alpha.-lactalbumin, LDH, lysozyme, myoglobin, ovalbumin, and RNase A. Stabilizers also include amino acids and their metabolites, such as, glycine, alanine (.alpha.-alanine, .beta.-alanine), arginine, betaine, leucine, lysine, glutamic acid, aspartic acid, proline, 4-hydroxyproline, sarcosine, γ-aminobutyric acid (GABA), opines (alanopine, octopine, strombine), and trimethylamine N-oxide (TMAO). In one embodiment the stabilizer is an amino acid.

In certain embodiments, the pharmaceutical formulation according to the present invention comprises a nonionic surfactant. Nonionic surfactants, include, but are not limited to, polyoxyethylensorbitan fatty acid esters (such as polysorbate 20 and polysorbate 80), polyethylene-polypropylene copolymers, polyethylene-polypropylene glycols, polyoxyethylene-stearates, polyoxyethylene alkyl ethers, e.g. polyoxyethylene monolauryl ether, alkylphenylpolyoxyethylene ethers (Triton-X), polyoxyethylene-polyoxypropylene copolymer (Poloxamer, Pluronic), sodium dodecyl sulphate (SDS). In one embodiment the nonionic surfactant is polysorbate 80. In one embodiment the polysorbate 80 concentration is about 0.005 to 0.02% (w/v). In one embodiment, the polysorbate 80 concentration is about 0.01% (w/v). In one embodiment, the polysorbate 80 concentration is about 0.02% (w/v).

In certain embodiments, the pharmaceutical formulation according to the present invention comprises a metal chelator. Metal chelators, include, but are not limited to EDTA and EGTA. In one embodiment the metal chelator is EDTA. In one embodiment the EDTA concentration is about 0.01 to about 0.02 mM. In one embodiment, the EDTA concentration is about 0.05 mM.

In one other aspect, the present invention is also directed to a method of treatment of a patient suffering from a disease caused by Hepatitis C virus in human or animal subjects, preferably for use in the treatment or prevention in human subjects, wherein the patient is administered an effective amount of the antibody or antigen-binding fragment thereof according to the invention, a nucleic acid of the invention or a pharmaceutical composition of the invention.

In another aspect, the present invention is also directed to the use of the antibody or antigen-binding fragment thereof according to the invention, a nucleic acid of the invention or a pharmaceutical composition of the invention in the manufacture of a medicament for treatment or prevention of a disease caused by Hepatitis C virus in human or animal subjects, preferably for treatment or prevention in human subjects.

All embodiments of the present invention as described herein are deemed to be combinable in any combination, unless the skilled person considers such a combination to not make any technical sense.

### Examples

### Virus particle production

HCV particles were produced as previously published (Koutsoudakis, G et al. (2006). J. Virol. 80, 5308-5320. and Wakita, T. et al. (2005). Nat. Med. 11, 791-796.).

Plasmids containing a T7-promoter, a Renilla luciferase gene, and genomes of the previously described (Bankwitz, D. et al. (2020). Gut gutjnl-2020-321190.) HCVcc strains **2r** (GT1b (J4) in Supplementary Table S1 of Bankwitz et al., 2020), **3a** (GT3a (S52) in Supplementary Table S1 of Bankwitz et al., 2020), **2a** (GT2a (J6) in Supplementary Table S1 of Bankwitz et al., 2020), **2a-3** (GT2a (2a-3) in Supplementary Table S1 of Bankwitz et al., 2020), **2b** (GT2b (J8) in Supplementary Table S1 of Bankwitz et al., 2020), **2b-4** (GT2b (2b-4) in Supplementary Table S1 of Bankwitz et al., 2020), **2b-5** (GT2b (2b-5) in Supplementary Table S1 of Bankwitz et al., 2020), **1b con** (GT1b (Con1) in Supplementary Table S1 of Bankwitz et al., 2020), **1a** (GT1a (H77) in Supplementary Table S1 of Bankwitz et al., 2020), **1b J4** (GT1b (J4) in Supplementary Table S1 of Bankwitz et al., 2020), **2k** (GT2k (2k) in Supplementary Table S1 of Bankwitz et al., 2020), **5a** (GT5a (SA13) in Supplementary Table S1 of Bankwitz et al., 2020), and **4a** (GT4a (ED43) in Supplementary Table S1 of Bankwitz et al., 2020) were used as templates for *in vitro* transcription with T7 RNA polymerase (Promega) in 80 mM HEPES pH 7.5, 12 mM MgCl₂, 3.125 mM (each) ribonucleotides (Roche), 2 mM spermidine (Sigma Aldrich), 40 mM DTT, and 1 U/µL RNasin (Promega) for 4 h at 37°C.

DNA was digested with DNase (Promega) for 30 min at 37°C. RNA was purified using an RNA cleanup kit (Machery Nagel). RNA was electroporated at 975 µF and 270 V into 6 x 10⁵ Huh 7.5.1 cells (Zhong et al., 2005), suspended in 400 µL Cytomix buffer (120 mM KCI, 0.15 mM CaCl₂, 10 mM K₂HPO₄/KH₂PO₄, 25 mM HEPES, 2 mM EGTA, 5 mM MgCl₂, pH 7.6). Cells were then cultured in DMEM (Gibco) supplemented with 2 mM L-glutamine (Gibco), 10% FBS (Capricorn), 1% Penicillin-Streptomycin (Gibco) and 1% MEM NEAA (Gibco) at 37°C and 5% CO₂. Supernatant was harvested after 48, 72, and 96 h, pooled, aliquoted, and stored at -80°C until further use.

### HCVcc neutralization assay

Neutralization assays were performed as previously described (Koutsoudakis, G et al. (2006). J. Virol. 80, 5308-5320. and Wakita, T. et al. (2005). Nat. Med. 11, 791-796.). In brief, Huh 7.5 cells (Blight, KJ. et al. (2002). J. Virol. 76, 13001-13014.) were seeded at a density of 10⁴ cells per well in a 96-well plate (Falcon) in 200 µl supplemented DMEM (see above) and incubated for 24 h at 37°C and 5% CO₂.

Virus strains used herein are 13 different renilla luciferase reporter HCVcc strains (2r, 3a, 2a, 2a-3, 2b, 2b-4, 2b-5, 1b con, 1a, 1b J4, 2k, 5a, and 4a) as previously disclosed in Bankwitz, D. et al. (2020). Gut gutjnl-2020-321190.

Equal volumes of viral supernatant and antibodies or purified IgG were mixed and co-incubated at 37°C for 30-60 min and 40 µL was added to precultured Huh 7.5 cells, from which the medium had been aspirated. After 4 h of culture, 160 µL supplemented DMEM (see above) was added to each well.

After 3 days of culture, supernatant was removed and cells were washed with PBS (Gibco) and lysed by adding 35 µL ddH₂O and freezing at -80°C. 20 µL of the thawed lysate was transferred to a white microtiter assay plate (Berthold). 60 µL of 0.424 µg/mL coelenterazine (PJK Biotech) in PBS was added to each well in a plate reader (Tri Star, Berthold). Renilla luminescence was determined (counting time 0.1 sec) after a reaction delay of 5 sec and shaking (1 sec).

Luminescence was quantified relative to wells infected with virus alone (at least 4 control wells on the same assay plate) to obtain % neutralization values. Neutralization above background was defined as reduction of luminescence by at least 40% based on neutralization values of an isotype control antibody (MGO-53) in 10 independent experiments.

### Expression and purification of E2 constructs

For ELISA experiments, Strep-tagged E2core proteins (residues 412 - 645) were expressed by transiently transfecting Expi293F cells (National Research Council of Canada) and purified from clarified supernatants via the Strep-tag.

### ELISA analysis to determine antibody binding activity to HCV E2 protein

96-well ELISA plates (Corning) were coated with 2.5 µg/mL protein in PBS over night at 4°C. After six times of washing with PBS-T (PBS, 0.05% v/v Tween-20), plates were blocked with 200 µL of 2% (w/v) bovine serum albumin (BSA, Carl Roth) in PBS, and incubated for 90 min at room temperature. Plates were then washed as before and horseradish peroxidase (HRP)-conjugated goat anti-human IgG antibody (Southern Biotech) was added (diluted 1:1000 in 2% w/v BSA/PBS) and incubated for 60 min at room temperature. Plates were then washed as before and ABTS solution (Thermo Fisher) was added. Readout was performed at an absorbance of 415-695 nm using a plate reader (Tecan Sunrise).

### Quantification and statistical analysis

Flow cytometry analysis and quantifications were done with FlowJo10. Statistical analyses were done with GraphPad Prism (v9), Microsoft Excel for Mac (v14.7.3), Python (v3.6.8), and R (v3.6.0).

A summary of the results obtained with the antibodies of the invention is shown in Figures 1 and 2. Reference antibodies used to raise the data presented in Figures 1 and 2 are HEPC3 and HEPC74 as described in Flyak et al., Cell Host Microbe. 2018 Nov 14;24(5):703-716.e3.doi: 10.1016/j.chom.2018.10.009., and AT12-007 and AT13-021 as described in published international patent application WO 2016/036252 A2.

### Method of treatment

Exemplary antibodies of the invention can be used for treatment of HCV-infection, the prevention of HCV-infection, or as post-exposure prophylaxis in individuals recently exposed to HCV. The antibodies of the invention can be provided as a single-use sterile solution at a concentration of 50 mg/mL. Each vial of an antibody drug product may contain 20 mL of a buffered solution composed of acetic acid, sodium acetate, glycine, trehalose, and polysorbate 20 (see table).

| **Component** | **Concentration [mg/mL]** |
|---|---|
| Antibody of the invention | 50.00 |
| Acetic acid glacial | 0.25 |
| Sodium acetate trihydrate | 2.15 |
| Glycine | 16.52 |
| Trehalose dihydrate | 7.57 |
| Polysorbate 20 | 0.40 |
| Water for Injection (WFI) | Ad 1 mL |

## Claims

1. Antibody or antigen-binding fragment thereof directed against Hepatitis C virus, wherein the antibody or antigen-binding fragment thereof comprises the heavy chain CDR1 to CDR3 and the light chain CDR1 to CDR3 amino acid sequence of one antibody from the group comprising 1416_01_E03 (with the CDR-H1 of SEQ ID NO: 1, CDR-H2 of SEQ ID NO: 2; CDR-H3 of SEQ ID NO: 3, CDR-L1 of SEQ ID NO: 4, CDR-L2 of SEQ ID NO: 5, and CDR-L2 of SEQ ID NO: 6), 1198_05_G10 (with the CDR-H1 of SEQ ID NO: 7, CDR-H2 of SEQ ID NO: 8; CDR-H3 of SEQ ID NO: 9, CDR-L1 of SEQ ID NO: 10, CDR-L2 of SEQ ID NO: 11, and CDR-L2 of SEQ ID NO: 12), 1382_01_H05 (with the CDR-H1 of SEQ ID NO: 13, CDR-H2 of SEQ ID NO: 14; CDR-H3 of SEQ ID NO: 15, CDR-L1 of SEQ ID NO: 16, CDR-L2 of SEQ ID NO: 17, and CDR-L2 of SEQ ID NO: 18), and 1334_03_A04 (with the CDR-H1 of SEQ ID NO: 19, CDR-H2 of SEQ ID NO: 20; CDR-H3 of SEQ ID NO: 21, CDR-L1 of SEQ ID NO: 22, CDR-L2 of SEQ ID NO: 23, and CDR-L2 of SEQ ID NO: 24).

2. Antibody or antigen-binding fragment thereof according to claim 1, wherein the antibody or antigen-binding fragment thereof comprises the combination of the variable region heavy chain amino acid sequence and of the variable region light chain amino acid sequence of one antibody selected from the group comprising 1416_01_E03 (with the variable region heavy chain amino acid sequence of SEQ ID NO: 25, and the variable region light chain amino acid sequence of SEQ ID NO: 26), 1198_05_G10 (with the variable region heavy chain amino acid sequence of SEQ ID NO: 27, and the variable region light chain amino acid sequence of SEQ ID NO: 28), 1382_01_H05 (with the variable region heavy chain amino acid sequence of SEQ ID NO: 29, and the variable region light chain amino acid sequence of SEQ ID NO: 30), and 1334_03_A04 (with the variable region heavy chain amino acid sequence of SEQ ID NO: 31, and the variable region light chain amino acid sequence of SEQ ID NO: 32).

3. Antibody according to any one of claims 1 or 2, wherein the antibody comprises the combination of the heavy chain sequence and the light chain sequence of one antibody selected from the group comprising 1416_01_E03 (with the heavy chain amino acid sequence of SEQ ID NO: 33, and the light chain amino acid sequence of SEQ ID NO: 34), 1198_05_G10 (with the heavy chain amino acid sequence of SEQ ID NO: 35, and the light chain amino acid sequence of SEQ ID NO: 36), 1382_01_H05 (with the heavy chain amino acid sequence of SEQ ID NO: 37, and the light chain amino acid sequence of SEQ ID NO: 38), and 1334_03_A04 (with the heavy chain amino acid sequence of SEQ ID NO: 39, and the light chain amino acid sequence of SEQ ID NO: 40).

4. Antibody or antigen-binding fragment according to any one of claims 1 to 3, wherein the CDR sequences, variable region sequences, or heavy chain and light chain sequences are taken from one antibody selected from the group comprising 1416_01_E03, 1198_05_G10, and 1382_01_H05, preferably from one antibody selected from 1416_01_E03 and 1198_05_G10.

5. Antibody or antigen-binding fragment according to any one of claims 1 to 4, wherein the CDR sequences, variable region sequences, or heavy chain and light chain sequences are taken from the antibody 1416_01_E03.

6. Pharmaceutical composition comprising an antibody or antigen-binding fragment thereof according to any one of claims 1 to 5 and at least one pharmaceutically acceptable excipient.

7. Pharmaceutical composition according to claim 6, wherein the pharmaceutical composition is a vaccination composition for a human or animal subject, preferably for a human subject.

8. Kit comprising an antibody or antigen-binding fragment thereof according to any one of claims 1 to 5 and a container.

9. Nucleic acid encoding an antibody or antigen-binding fragment thereof as defined in any of claims 1 to 5.

10. An expression vector comprising the nucleic acid of claim 9 in functional association with an expression control sequence.

11. Host cell comprising a nucleic acid according to claim 9 or the expression vector according to claim 10.

12. Method of production of an antibody or antigen-binding fragment thereof of any one of claims 1 to 5, comprising
(a) cultivating the host cell of claim 11 under conditions allowing expression of the antibody or antigen-binding fragment thereof, and
(b) recovering the antibody or antigen-binding fragment thereof.

13. Antibody or antigen-binding fragment thereof according to any one of claims 1 to 5, pharmaceutical composition according to claim 6 or 7, kit according to claim 8, nucleic acid according to claim 9, or expression vector according to claim 10 for use as a medicament.

14. Antibody or antigen-binding fragment thereof according to any one of claims 1 to 5, pharmaceutical composition according to claim 6 or 7, kit according to claim 8, nucleic acid according to claim 9, or expression vector according to claim 10 for use in the treatment or prevention of a disease caused by Hepatitis C virus in human or animal subjects or for use in prevention of infection of a human or animal subject with Hepatitis C virus, preferably for use in human subjects.

15. Antibody or antigen-binding fragment thereof according to any one of claims 1 to 5, pharmaceutical composition according to claim 6 or 7, kit according to claim 8, nucleic acid according to claim 9, or expression vector according to claim 10 for use according to claim 14, wherein the use comprises passive immunization of the human or animal subject.
